# EUROPEAN PATENT APPLICATION

(11) **EP 1 790 235 A1**
(43) Date of publication of application: **30.05.2007**
(21) Application number: 05770591.5
(22) Date of filing: 08.08.2005
(51) Int. Cl.: A23L 1/00, A61K 9/50, A61K 31/23, A61K 31/231, A61K 47/02, A61K 47/36

(54) **METHOD FOR PRODUCING CAPSINOID-CONTAINING MICROCAPSULE**

(30) Priority: 11.08.2004 JP 2004234472; 11.08.2004 JP 2004234471
(71) Applicant: Ajinomoto Co., Inc., Tokyo 104-8315 (JP)
(72) Inventor: TAKAKURA, Yukiko, -ku, Kawasaki-shi, Kanagawa 2108681; (JP); MARUYAMA, Kentarou, -ku, Kawasaki-shi, Kanagawa 2108681; (JP)
(74) Representative: Strehl Schübel-Hopf & Partner
(86) International application number: PCT/JP2005/014868
(87) International publication number: WO 2006/016685

(57) **Abstract**

[Problem] To provide capsinoid-containing microcapsules.

[Solution] Capsinoid-containing microcapsules comprising 0.01 to 10 weight parts of capsinoid-containing hydrophobic material per weight part of hydrophilic solution containing 0.001 to 50 percent of capsule wall material and having a median diameter of 0.01 to 1,000 µm are provided.

## Description

### TECHNICAL FIELD

The present invention relates to capsinoid-containing microcapsules and to a method for manufacturing the same.

### BACKGROUND ART

As a chili pepper with little pungency, the non-pungent fixed species "CH-19 Sweet" of chili pepper selectively fixed by Yazawa et al. contains almost none of the pungent, invasive capsaicinoid compounds (capsaicin, dihydrocapsaicin, and the like; sometimes referred to as "capsaicinoids" hereinafter) of common chili peppers. However, it is reported to contain large quantities of novel, non-pungent capsinoid compounds (fatty acid esters of vanillyl alcohol, capsiates, dihydrocapsiates, and the like; sometimes referred to simply as "capsinoids" hereinafter) (Patent Reference 1). Further, these capsinoids have been identified in other plants of the genus *Capsicum* (Nonpatent Reference 1).

Since the molecular structure of capsinoids contains ester bonds, they are unstable in the presence of water. They are also characterized by decomposing extremely readily in the presence of heat. Accordingly, when employed in foods, in actual industrial production, it is necessary to prevent the decomposition of capsinoids and ensure their stability.

Patent Reference 2 proposes a emulsified capsinoid employed as an emulsifier for fatty acid monoglycerides and polysaccharides (Patent Reference 2). However, applications are limited because the pH range over which emulsifiers can be employed is limited. This product thus lacks adequate practicality. Further, Patent Reference 3 proposes a capsinoid-containing microcapsule comprising capsaicinoids as representative components (Patent Reference 3). However, as stated above, since capsinoids differ from capsaicinoids in the form of the bond between the vanillyl alcohol and fatty acid, their physical properties - such as resistance to hydrolysis - differ greatly. Thus, it is difficult to employ a technique suited to capsaicinoids directly on capsinoids and achieve stability.

A variety of capsules are employed to stabilize food components. Despite the variety found in capsules, soft capsules and hard capsules such as tablets have a diameter of 1 to 2 cm, a size that can be detected by the tongue during ingestion. The encapsulation and use in foods of capsinoids is thus problematic.
[Patent Reference 1] Japanese Patent Application Publication No. Heisei 11-246478
[Patent Reference 2] Japanese Patent Application Publication No. 2003-192576
[Patent Reference 3] Japanese Patent Application Publication No. 2003-47432
[Nonpatent Reference 1] Journal of the Japanese Society for Horticultural Science 58, p. 601-607.

### DISCLOSURE OF THE INVENTION

The object of the present invention is to provide a method for manufacturing capsinoid-containing microcapsules and a capsinoid-containing microcapsule permitting the stable, industrial use of capsinoids.

As a result of extensive research into achieving the above object, the present inventors stabilized capsinoids by microencapsulation. More specifically, a capsinoid-containing microcapsule that could be advantageously used in industrial production was devised using a microencapsulation technique that is simpler than those employed in the manufacturing of conventional, known microcapsules. That is, the present invention relates to a method for manufacturing a capsinoid-containing microcapsule by which a capsinoid-containing microcapsule is readily manufactured by coacervation, ultrasonic waves, interfacial precipitation, spray drying, the orifice method, or the like. More particularly, the present invention relates to a method for manufacturing a capsinoid-containing microcapsule in which the capsule wall material is comprised of one or more selected from the group consisting of polysaccharides, proteins, and polyamino acids, and a method for manufacturing a capsinoid-containing microcapsule in which the capsule wall does not contain a reinforcing agent or emulsifier. That is, it was discovered that capsinoid-containing microcapsules with a median diameter of 0.01 to 1,000 µm could be manufactured by ultrasonic treatment for 0.1 second to 60 minutes in a mixed solution comprising 0.01 to 10 weight parts of hydrophobic material per weight part of hydrophilic solution containing 0.001 to 50 percent of capsule wall material. The present invention was devised on this basis. It was further discovered that microcapsules with a median diameter of 0.01 to 1,000 µm could be manufactured by preparing an O/W emulsion with an aqueous phase containing pectin, a non-animal material, and an oil phase, and then admixing polyvalent cations to encapsulate the capsinoid-containing hydrophobic material. The present invention was also devised on this basis.

That is, the present invention relates to capsinoid-containing microcapsules (sometimes referred to simply as "microcapsules" hereinafter) and covers the following:
[1] A capsinoid-containing microcapsule with a median diameter of 0.01 to 1,000 µm, comprising 0.01 to 10 weight parts of a capsinoid-containing hydrophobic material per weight part of a hydrophilic aqueous solution containing 0.001 to 50 percent of a capsule wall material.
[2] The microcapsule according to claim 1 wherein said capsule wall material is one or more members selected from the group consisting of polysaccharides, proteins, and polyamino acids.
[3] The microcapsule according to claim 1 wherein said capsule wall material is one or more members selected from the group consisting of alginic acid, pectin, and BSA.
[4] The microcapsule according to any of [1] to [3], containing a pectin polyvalent cationic gel in the wall thereof, encapsulating a capsinoid-containing hydrophobic material, and having a median diameter of 0.01 to 100 µm, obtained by preparing an O/W emulsion with an aqueous phase containing 0.01 to 10 weight percent pectin and an oil phase and then admixing polyvalent cations.
[5] The microcapsule according to [4] wherein said polyvalent cations are calcium ions.
[6] The microcapsule according to [4] or [5], further characterized in that no emulsifier is employed when preparing the O/W emulsion.
[7] The microcapsule according to any of [4] to [6], further characterized in that the pectin comprises an amide group.
[8] The microcapsule according to any of [4] to [7], further characterized in that the hydrophobic material containing the encapsulated capsinoid is a W/O emulsion.
[9] The microcapsule according to any of [1] to [8], further characterized in that pectin methyl esterase is added after preparing said O/W emulsion and admixing polyvalent cations.
[10] The microcapsule according to [9], further characterized in that polyvalent cations are added after adding said pectin methyl esterase.
[11] The microcapsule according to any of [1] to [10], further characterized in that said hydrophobic material containing the capsinoids is one or more selected from the group consisting of capsiates, dihydrocapsiates, or nordihydrocapsiates.
[12] A method for manufacturing the microcapsule according to any of [1] to [3] with a median diameter of 0.01 to 1,000 µm, comprising ultrasonically treating for from 0.1 second to 60 minutes a mixed solution of 0.01 to 10 weight parts of capsinoid-containing hydrophobic material per weight part of hydrophilic solution containing 0.001 to 50 percent of capsule film material;.
[13] The method according to [12] wherein said capsule film material is alginic acid and calcium chloride is employed as a reinforcing agent.
[14] The method according to [12] further characterized in that the capsule film material does not contain a reinforcing agent.
[15] The method according to [12] further characterized in that the capsule film material does not contain an emulsifier.
The present invention further covers the following capsinoid-containing microcapsules: 1) microcapsules that are obtained by preparing an O/W emulsion with an aqueous phase containing 0.01 to 10 weight percent pectin and an oil phase and then admixing polyvalent cations, contain a pectin polyvalent cation gel in the wall, encapsulate a hydrophobic material containing capsinoids, and have a median diameter of 0.01 to 1,000 µm; 2) microcapsules that are obtained by preparing an O/W emulsion with an aqueous phase containing 0.01 to 10 weight percent pectin and an oil phase and then admixing calcium ions, contain a pectin calcium gel in the wall, encapsulate a hydrophobic material containing capsinoids, and have a median diameter of 0.01 to 1,000 µm; 3) the microcapsules of 1) or 2) further characterized in that an emulsifier is not employed in the preparation of the O/W emulsion; 4) the microcapsule of any of 1) to 3) further characterized in that the pectin has an amide group; 5) the microcapsules of any of 1) to 4) further characterized in that the encapsulated hydrophobic material containing capsinoids is a W/O emulsion; 6) the capsinoid-containing microcapsule of any of 1) to 5) further characterized in that an O/W emulsion is prepared, cationic anions are admixed, and pectin methyl esterase is added; and 7) the capsinoid-containing microcapsule of 6) further characterized in that after adding said pectin methyl esterase, polyvalent cations are added.

### EFFECT OF THE INVENTION

Specifically, the present invention provides capsinoid-containing microcapsules having a median diameter of 0.01 to 1,000 µm, and a method for manufacturing the same, by ultrasonically treating for from 0.1 second to 60 minutes a mixed solution of 0.01 to 10 weight parts of capsinoid-containing hydrophobic material per weight part of a hydrophilic solution containing 0.001 to 50 percent of a capsule wall material. The present invention further provides capsinoid-containing microcapsules in which the encapsulated material is hydrophobic; the capsule wall is pectin, a non-animal product; and no surfactant or emulsifier is required as the capsule material; that are readily prepared, afford good stability, and encapsulate a large quantity of oil per capsule.

### BRIEF DESCRIPTION OF THE DRAWINGS

Fig. 1: Particle size distribution of pectin microcapsules.
Fig. 2: Particle size distribution of alginic acid microcapsules.

### BEST MODE OF IMPLEMENTING THE INVENTION

In the present invention, the term "capsinoids" refers to the fatty acid esters of vanillyl alcohols that are contained as components in non-pungent chili peppers, typified by capsiates, dihydrocapsiates, and nordihydrocapsiates.

Capsinoids are contained in large quantity in plants of the genus *Capsicum.* They can thus be prepared by purification and separation from the body and/or fruit of plants of the genus *Capsicum* (referred to hereinafter as "chili peppers"). The capsinoid-containing chili peppers employed may be derived from local pungent chili pepper species typified by "Nikko," "Goshiki," and the like, or may be any other type of chili pepper that contains capsinoids. Of these, local non-pungent species of chili peppers typified by "CH-19 Sweet," "Manganji," "Fushimi Amanaga," "Shishito" peppers, and green peppers contain many capsinoids and are suitably employed. The non-pungent species "CH-19 Sweet" contains a large quantity of such components and is thus employed with particular preference. Here, the term "CH-19 Sweet" includes both "CH-19 Sweet" and subsequent-generation analog species of "CH-1 9 Sweet". In the present Specification, the term "CH-19 Sweet" is employed to mean all of these. Purification and separation may be conducted by solvent extraction, various forms of chromatography such as silica gel chromatography, and means such as preparation-use high-performance chromatography, all of which are well-known to those skilled in the art. These techniques may be employed singly or in suitable combinations. For example, the method described in Japanese Patent Application Publication No. Heisei 11-246478 may be employed.

Further, the above capsinoids may be synthesized by an esterification reaction employing starting materials in the form of reactive fatty acid esters and vanillyl alcohol, as is described for example in Japanese Patent Application Publication No. Heisei 11-246478. Alternatively, based on structural formula, they may be synthesized by other reaction processes known to those skilled in the art. Still further, they may be readily prepared by a synthesis method employing enzymes. For example, the method described in Japanese Patent Application Publication No. 2000-312598 may be used to obtain a desired capsinoid compound by a reverse reaction with lipase employing vanillyl alcohol as substrate from a compound such as a fatty acid ester corresponding to the desired compound and/or a triglyceride containing a particular fatty acid.
In the present invention, the term "microcapsule" refers to capsules having a median diameter of 0.01 to 1,000 µm. A small median diameter is desirable from the perspectives of the safety of the capsule and avoiding detection by people when ingested. To avoid detection by people during ingestion, a median diameter of 100 µm, preferably 10 µm or less, and more preferably, 3 µm or less is desirable. Methods of manufacturing capsules include seamless capsule manufacturing methods employing multiple tube nozzles, manufacturing methods based on coacervation, and methods of manufacturing capsules based on tablet making. The present invention is characterized by the use of ultrasonic waves, permitting the manufacturing of uniform capsules of small median diameter. In the present invention, capsinoids are encapsulated as active components in the microcapsules. Thus, from the perspective of encapsulation efficiency, a median diameter of 0.1 to 1,000 µm is desirable, and from the perspective of mouthfeel during ingestion, a median diameter of about 0.01 to 100 µm is desirable.
In manufacturing capsules by the present invention, it suffices to employ a method capable of manufacturing capsules with a median diameter of 0.01 to 1,000 µm, preferably 0.01 to 100 µm. Examples of such methods are the coacervation method, interfacial precipitation, spray drying, the orifice method (seamless capsule method), coating method, and extrusion method.

The material of the capsule wall in the present invention can be any hydrophilic polymer. When the capsule is employed as a food material, edible materials, particularly polysaccharides, proteins, and polyamino acids, are employed with preference. More specifically, the use of one or more materials selected from the group consisting of polyglutamic acid ("PGA" hereinafter), alginic acid, carrageenan, pectin, and mixed gelatin-gum arabic systems is desirable to enhance the safety of the microcapsule of the present invention. Of these, alginic acid, pectin, and BSA are particularly preferred compounds. Pectin is the capsule wall material of greatest preference. Other materials suitable for use as the wall are polyacrylic acid, cellulose ethers, cellulose esters, and various other polymers. When employing an edible material, the capsinoid-containing microcapsule can be mixed with a food or the like for use.
The concentration of the capsule wall material in the hydrophilic solution in the present invention is desirably 0.001 to 50 percent, preferably 0.005 to 20 percent, and more preferably 0.01 to 2 percent. Concentrations of the capsule wall material that are above or below this range undesirably cause the particle size to increase and reduce the number of capsules. When employing pectin as the capsule wall material, the concentration of pectin in the aqueous solution is desirably 0.01 to 10 percent, preferably 0.1 to 3 percent, and more preferably 1 to 3 percent. An excessively high pectin concentration causes the capsules to undesirably aggregate, while an excessively low pectin concentration is undesirable in that safety is low and encapsulated oil tends to separate. The pectin employed as capsule wall is desirably amidated to increase the strength of the gel through hydrophobic bonds.

During ultrasonic treatment, the capsinoid-containing solution in the form of a hydrophobic material that is mixed with a hydrophilic solution containing the capsule wall material may be a hydrophobic solution or a hydrophobic solid. From the perspectives of handling, cost, capsinoid stability, and safety, an edible oil is desirable. From 0.01 to 10 weight parts of hydrophobic material per weight part of hydrophilic material suffices. However, one or fewer weight parts of hydrophobic material per weight part of hydrophilic material is desirable because the capsule contents tend to become hydrophobic; by mixing capsinoids into the hydrophobic material in advance, it is possible to manufacture a microcapsule containing capsinoids all at once, without having to separately manufacture the microcapsule and its contents. In the present Specification, unless specifically stated otherwise, examples of hydrophobic materials into which capsinoids have been mixed are described for the contents of the microcapsule.
In the present invention, the microcapsule can be prepared by a variety of methods, such as the coacervation method, interfacial precipitation, spray drying, orifice method (seamless capsule method), coating method, and extrusion method. In one form, the microcapsules are manufactured by an ultrasonic treatment lasting 0.1 seconds to 60 minutes. Here, the intensity of the ultrasonic treatment is not particularly limited so long as microcapsules form. However, an ultrasonic wave intensity of 1 to 10,000 W, preferably 10 to 2,000 W, is normally desirable. An excessively high ultrasonic wave intensity is undesirable because capsules end up being broken by the excessive energy, reducing the number of capsules. An excessively low ultrasonic wave intensity is undesirable because adequate emulsification does not take place, increasing the particle size.
Further, when forming microcapsules by the above-described ultrasonic treatment, the minimum ultrasonic treatment period is 0.1 second or more, preferably 20 seconds or more, and more preferably 30 seconds or more. An excessively short treatment period is undesirable because adequate emulsification does not take place, increasing the particle size. The maximum ultrasonic treatment period is 60 minutes or less, preferably 20 minutes or less, and more preferably two minutes or less. An excessively long ultrasonic treatment period is undesirable because capsules end up being broken by the excess energy, reducing the number of capsules.

The temperature of the ultrasonic treatment is not specifically limited so long as microcapsules form. However, 4 to 100°C is desirable and 10 to 60°C is preferred. An excessively high temperature is undesirable because it ruptures the capsules. An excessively low temperature is undesirable because not only does the solution freeze, but the solution viscosity becomes excessively high, tending to prevent transmission of emulsification energy and tending to prevent capsule formation.
The ultrasonic treatment need not be conducted continuously; so long as microcapsules form, it may be conducted intermittently.
Generally, processes in which alum, calcium chloride, potassium chloride, and other reinforcing agents are added to stabilize the capsules are known. In the present invention, as well, stronger microcapsules can be formed by adding a reinforcing agent. However, when forming microcapsules by the above-described ultrasonic treatment, for reasons such as ensuring safe consumption in food and pharmaceuticals, odor, and cost, the fact that microcapsules can be stably formed without incorporating reinforcing agents into the capsule wall material is characteristic.
In the present invention, the term "reinforcing agent" means a substance that generates cations in solution. Examples of such additives are metal salts such as alum, calcium chloride, and potassium chloride. These metal salts become cations in solution, bond to the capsule wall material, and reinforce the capsule wall.

Reinforcing agents are not required for the formation of microcapsules in the present invention. However, when adding reinforcing agents to reinforce the capsules, the concentration is normally 2 M or less, preferably 0.2 M or less. An excessively high concentration of reinforcing agent is undesirable because the particle size becomes excessively large and the capsules tend to aggregate.
When employing pectin as the capsule material, after preparing an O/W emulsion with a pectin-containing aqueous phase and an oil phase, it is important to admix polyvalent cations. From the perspective of greater stability, the polyvalent cations are desirably calcium ions. In this process, the concentration of the calcium ions is 0.01 to 1,000 mM, desirably 0.01 to 100 mM, preferably 1 to 10 mM. An excessively high concentration of polyvalent cations is undesirable from the perspective of capsule aggregation, while an excessively low concentration of polyvalent cations is undesirable in that capsule stability decreases and encapsulated oil separates.
As an example of the present invention, denoting the relative volume of the mixed solution of hydrophilic solution and hydrophobic material as 1, a reinforcing agent with a relative volume of 0.1 or less can be admixed using a device such as a microsyringe pump. This forms microcapsules of greater stability. An excessively high rate of addition of reinforcing agent is undesirable in that aggregation tends to occur.
Generally, the addition of an emulsifier is known when preparing an emulsion or suspension. In the present invention, as well, an emulsifier such as a sugar ester, monoglyceride, or sorbitan ester can be added to form microcapsules. However, when forming microcapsules by the above-described ultrasonic treatment, for reasons such as ensuring safe consumption in food and pharmaceuticals, odor, and cost, the fact that microcapsules can be stably formed without incorporating emulsifiers into the capsule wall material is characteristic.
When forming microcapsules by the above-described ultrasonic treatment, the fact that microcapsules can be manufactured in a single stage, without requiring multiple-stage operations in the manufacturing process, is characteristic. That is, this treatment is particularly efficient in industrial manufacturing because a preliminary emulsification step with a stirrer and a dropwise capsule formation step are not required.

The microcapsules can be separated by the usual separation operations, such as filtration and dialysis. Powders can be obtained by freeze-drying, spraying, and pressure reduction operations. When employing pectin as the capsule wall, the structural stability to heating loads and encapsulated material stability of the capsules are enhanced by reaction with pectin methyl esterase (sometimes referred to as "PME" hereinafter) following capsule preparation. Adding polyvalent cations, preferably calcium ions, to the PME further enhances the structural stability to heating loads and encapsulated material stability of the capsules. The PME concentration is 2 to 300 mPEU/mL, desirably 20 to 300 mPEU/mL. An excessively high PME concentration is undesirable because the capsules tend to aggregate. An excessively low PME concentration is undesirable because there is little increase in stabilizing effect. Here, the term "PEU" is used as an abbreviation of "pectin esterase unit" It is a unit indicating the ability of 1 mL of PME to decompose methyl ester for one minute and produce 1 mmol of acid.
Although it is not necessary to add polyvalent cations, specifically calcium ions, to PME, when calcium ions are added to increase the stability to heating loads, the concentration is 0.01 to 20 mM, preferably 1 to 10 mM. An excessively high concentration of calcium ions is undesirable because the capsules aggregate. An excessively low concentration of calcium ions is undesirable because there is little increase in the stabilizing effect. When calcium ions are not added, free carboxyl groups that are not chelated by the calcium ions become excessive, causing the capsules to aggregate and weakening the capsule wall structure.

When conducting a PME treatment prior to capsule preparation, overall gelling occurs during capsule preparation and capsules do not form. Thus, PME treatment and the addition of calcium chloride are conducted after capsule preparation to further reinforce the capsule walls.
The present invention is described below based on embodiments. However, the scope of the technique of the present invention is not limited to these embodiments. Unless specifically stated otherwise, the percentages given in the present invention denote weight percentages.

### EMBODIMENTS

The present invention is described below based on embodiments. However, the scope of the technique of the present invention is not limited to these embodiments. Unless specifically stated otherwise, the percentages given in the present invention denote weight percentages.

### (Embodiment 1)

### Examination of the microcapsule wall

The following combinations of microcapsule wall and cation were employed.
1) Pectin 0.02 to 5 percent, calcium chloride 0 to 700 mM
2) Alginic acid 0.25 to 1 percent, calcium chloride 0 to 0.1 M
3) PGA 1 to 40 percent, alum 2 to 200 mM
4) κ-Carrageenan 0.1 to 1 percent, calcium chloride 0 to 0.2 M
5) τ-Carrageenan 0.1 to 1 percent, calcium chloride 0 to 0.2 M
6) Gelatin - gum arabic (1 to 1 mixture) 0.01 to 0.1 percent
Optimal conditions were examined for each of these combinations, and the following were determined:
1) Pectin 2 percent, calcium chloride 7 mM
2) Alginic acid 0.25 percent, calcium chloride 0.05 M
3) PGA 1 percent, alum 5 mM
4) κ-Carrageenan 0.5 percent, calcium chloride 0.02 M
5) 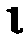 -Carrageenan 0.5 percent, calcium chloride 0.2 M
6) Gelatin - gum arabic 0.01 percent
   Each of the wall solutions and 3 mL of soybean oil (made by Ajinomoto Oil Mills Co., Ltd.) were charged to 50 mL stainless-steel tubes. While cooling the exterior of the stainless-steel tubes with ice, an ultrasonic processor, the Sonifier 250 (made by Branson Corp.), was used to conduct a two-minute treatment at an output of 148 W. When 30 seconds had elapsed after the start of the ultrasonic treatment, an IC 3100 microsyringe pump (made by KD Scientific Corp.) was used to add various cationic solutions at a rate of 127 mL/h, yielding milk-white dispersions.
   The oil encapsulation rate (%) of each of the microcapsules (each sample was centrifuged to separate the oil that had not been encapsulated to measure the quantity of encapsulated oil, the percentage given being the ratio of encapsulated oil to the total quantity of oil; oil content was determined by the Soxhlet method), the median diameter (µm), and the number of capsules (x 1012/L) were measured. The results are given in Table 1.

**[Table 1]**

| No. | 1 | 2 | 3 | 4 | 5 | 6 |
|---|---|---|---|---|---|---|
| Wall | Pectin 2% | Alginic acid 0.25% | PGA 1% | κ-Carrageenan 0.5% | 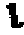 -Carrageenan 0.5% | Gelatin-gum arabic 0.01% |
| Oil encapsulation rate (%) | 97 | 94 | 16 | 6 | 4 | 26 |
| Median diameter (µm) | 1.1 | 10 | 3.3 | 2.8 | 3.5 | 2.6 |
| No. of capsules (× 10¹²/L) | 150 | 6.4 | 0.54 | 0.45 | 2.8 | 5.9 |

From Table 1, it will be understood that PGA, κ-carrageenan, 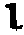 -carrageenan, and gelatin-gum arabic had low oil encapsulation rates of 30 percent or less. However, pectin had a desirably high oil encapsulation rate of 97 percent and alginic acid had a desirably high oil encapsulation rate of 94 percent. In particular, pectin also had a desirably low median diameter of 1 µm and a desirably high number of capsules, at 150 x 10¹²/L. Pectin was thus found to be a promising microcapsule material.

### (Embodiment 2)

### Manufacturing of pectin capsules with an ultrasonic device

Pectin (product name "LM-104AS", made by CPKelco JAPAN) and deionized water were mixed to prepare a 2 percent pectin aqueous solution. Next, 27 mL of 2 percent pectin aqueous solution and 3 mL of soybean oil (made by Ajinomoto Oil Mills Co., Ltd.) were added to a 50 mL stainless-steel tube. While cooling the exterior of the stainless-steel tube with ice, an ultrasonic processor, the Sonifier 250 (made by Branson Corp.), was used to conduct a two-minute treatment at an output of 145 W. When about 30 seconds had elapsed after the start of ultrasonic processing, an IC 3100 microsyringe pump (made by KD Scientific Corp.) was used to add 2 mL of 7.3 mM calcium chloride at a rate of 127 mL/h, yielding a milk-white dispersion.

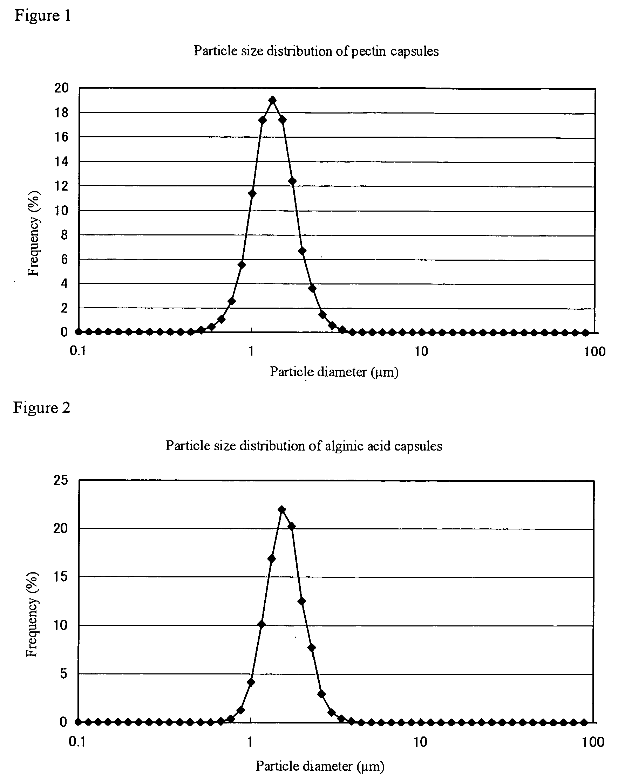

The above dispersion, in which the soybean oil had been fluorescent dyed with Nile Red and the pectin with Rhodamine, was observed with an LSM510 confocal laser scanning microscope (made by Carl Zeiss Corp.) to confirm the formation of microcapsules encapsulating soybean oil. Measurement of the particle size distribution of the microcapsule dispersion with an LA920 laser light-scattering particle size distribution analyzer (made by Horiba Instruments) revealed a single peak, with the median outer diameter of the microcapsules being 1.3 µm (Fig. 1).

### (Embodiment 3)

### Manufacturing of microcapsules with a high shear processor

Pectin (product name "LM-104AS", made by CPKelco JAPAN) and deionized water were mixed to prepare a 2 percent pectin aqueous solution. Next, 81 mL of 2 percent pectin aqueous solution and 9 mL of soybean oil (made by Ajinomoto Oil Mills Co., Ltd.) were added to the stirring vessel of a high shear processor (special order unit made by Nissei Corporation). While cooling the exterior of the vessel with ice, processing was conducted by the high shear processor for 3 minutes at 9,500 rpm. When about one minute had elapsed after the start of stirring, 6 mL of 7.3 mM calcium chloride was added over one minute, yielding a milk-white dispersion.
Measurement of the particle size distribution of the microcapsule dispersion with an LA920 laser light-scattering particle size distribution analyzer (made by Horiba Instruments) revealed a single peak, with the median outer diameter of the microcapsules being 4.1 µm.

### (Embodiment 4)

### The effects of pectin concentration and calcium concentration on the particle size of the microcapsules

Employing varied pectin (product name "LM-104AS", made by CPKelco JAPAN) concentrations of 0.02 percent, 0.2 percent, 2 percent, and 5 percent, microcapsules were prepared by the same method as in Embodiment 2.
To each of the individual pectin concentrations, 2 mL of calcium chloride was added after 30 seconds at varied concentrations of 0 mM, 7 mM, 70 mM, and 700 mM.
Table 2 gives the particle sizes of the various microcapsules. Unless specifically stated otherwise, the numbers given in the table denote median diameters (µm). The term "aggregation" was employed to denote cases of intense gelling where the sample becomes a single block, precluding the measurement of the particle size distribution.

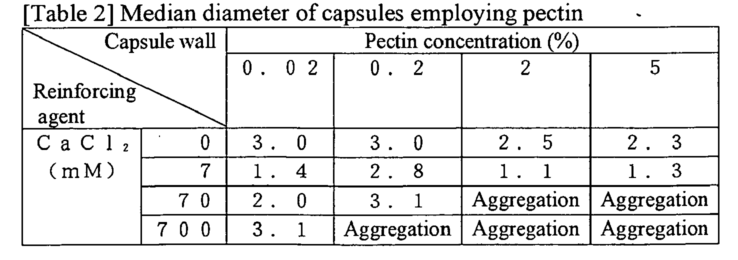

As shown in Table 2, as the pectin concentration and calcium chloride concentration increased, the particles tended to aggregate. The least aggregation occurred under conditions of 2 percent pectin and 7 mM calcium chloride, yielding capsules of low median diameter.

### (Embodiment 5)

### Evaluation of microcapsule stability by degree of pectin esterification and the presence or absence of amide groups

As shown in Table 3 below, microcapsules were prepared by the same method as in Embodiment 1 using various pectins of types LMA and LMC of varying degrees of esterification (DE values) of 31, 34, and 36. The microcapsules that were prepared were heat treated for 30 minutes at 120°C and evaluated for stability before and after the heat treatment.

**[Table 3] Table of degrees of esterification and stability of pectins**

| Product name | DE value | Type |
|---|---|---|
| 104AS (made by CPKelco Japan) | 31 | LMA (amidated) |
| 102AS (made by CPKelco Japan) | 34 | LMA (amidated) |
| 101AS (made by CPKelco Japan) | 36 | LMA (amidated) |
| 12CG (made by CPKelco Japan) | 31 | LMC |
| 13CG (made by CPKelco Japan) | 38 | LMC |

| | | |
|---|---|---|
| LMA: Low methoxyl amidated pectin LMC: Low methoxyl conventional pectin | | |

Table 4 shows the median diameter and number of capsules before and after heating. Following preparation, the microcapsule dispersions were all unseparated white suspensions. After heating, the LMA (amidated type) was a stable dispersion, but both 12CG and 13CG, which were LMC, separated.

**[Table 4] Changes in median diameter and number of capsules before and after heating**

| Pectin | Median diameter (µm) | | No. of capsules (x 10¹³/Liter) | |
|---|---|---|---|---|
| | Before heating | After heating | Before heating | After heating |
| 104AS | 0.9 | 2.7 | 18.1 | 5.9 |
| 102AS | 1.0 | 3.6 | 16.3 | 5.2 |
| 101AS | 1.1 | 1.6 | 15.9 | 9.9 |
| 12CG | 2.1 | 15.1 | 4.7 | 0.1 |
| 13CG | 2.5 | 11.9 | 4.2 | 0.3 |

As shown in Table 4, all the microcapsules aggregated or clumped after heating, increasing the median diameter. However, for LMA pectin, the higher the degree of esterification, the lower the median diameter. 101AS, with a DE value of 36, exhibited a low change in median diameter of 1.5-fold and a particle size distribution which remained a single peak, thus forming the capsules with the greatest structural stability. LMC pectin aggregated or clumped severely with heating, exhibiting an increased median diameter, and was thus found unsuited to the preparation of microcapsules.

### (Embodiment 6)

### The effects of pectin methyl esterase and the calcium concentration on microcapsule stability

A 5 mL quantity of microcapsule dispersion prepared by the same method as in Embodiment 2 and 5 mL of PME solution of varying concentrations were admixed and reacted in a vibrator for four hours at a temperature of 40°C at pH 4. Subsequently, 1 mL of calcium chloride solution of varying concentrations was added. The concentration of mixed PMEs (product name: NOVO SHAPE, made by Novozyme) were 0, 2.5, 25, 250, and 500 PEU/mL and the concentrations of calcium chloride added were 7, 10, 15, 25, and 450. The results are given in Table 5.

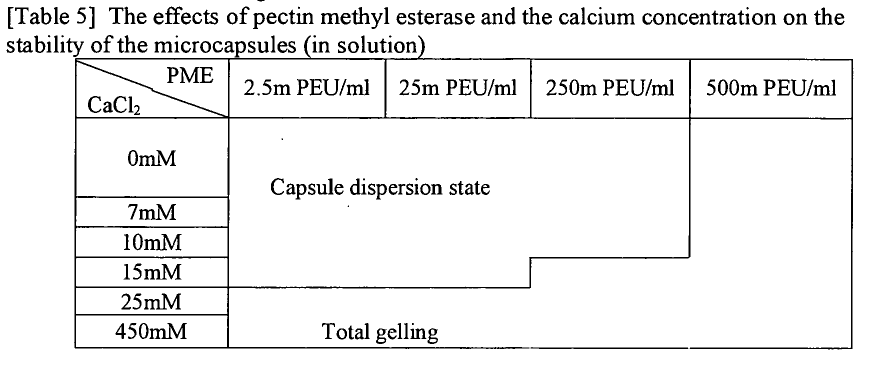

It will be understood from Table 5 that total gelling occurred at a PME concentration of 500 mPEU/mL at all calcium chloride concentrations, at a PME concentration of 250 mPEU/mL at calcium chloride concentrations of 15 mM and above, and at other PME concentrations at calcium chloride concentrations of 25 mM and above.

### (Embodiment 7)

### The effect on heating resistance of the addition of calcium chloride following PME treatment

PME solutions of varying concentrations were mixed with microcapsule dispersions manufactured by the same method as in Embodiment 2. Subsequently, samples to which 1 mL of 7 mM calcium chloride was added and samples to which no calcium chloride was added were prepared (all pH 4). These were heated for 30 minutes at 120°C, after which the median diameters before and after heating were measured. The concentration of the mixed PMEs (product name: NOVO SHAPE, made by Novozyme Corp.) were 0. 2.5, 25, and 250 mPEU/mL. The results are given in Table 6.
Microcapsules prepared by the method of Embodiment 2 were employed as a control.

**[Table 6] Median diameter (µm) before and after heating based on the addition of calcium chloride**

| | Control | 0 mPEU/mL | 2.5 m PEU/mL | 25 mPEU/mL | 250 mPEU/mL |
|---|---|---|---|---|---|
| With Ca | 1. 4 | 1. 9 | 2. 7 | 3. 5 | 4. 2 |
| Without Ca | 1. 4 | 1. 4 | 1. 4 | 1. 4 | 1. 4 |

From Table 6, it will be understood that when no calcium chloride was added following PME treatment, the greater the PME concentration, the more clumping and aggregation that occurred following heating and the greater the median diameter. However, when calcium chloride was added following PME treatment, the median diameter was maintained even after heating. Based on these results, when PME treatment is conducted, the addition of calcium chloride following PME treatment imparts resistance to heating loads to the capsule structure. Within the PME concentration range of 0 to 250 mPEU/mL, capsules in which calcium chloride was added to 50 mPEU/mL of PME exhibited the greatest stability and were preferred.

### (Embodiment 8)

### Manufacturing of capsinoid-containing microcapsules with an ultrasonic device

Pectin (product name "LM-104AS", made by CPKelco JAPAN) and deionized water were mixed to prepare a 2 percent pectin aqueous solution. Next, 27 mL of the 2 percent pectin aqueous solution and 3 mL of soybean oil (made by Ajinomoto Oil Mills) containing 2 percent capsinoids (with a purity based on capsinoids of 97.5 percent and a capsiate to dihydrocapsiate ratio of about 2:1) were charged to a 50 mL stainless steel tube. While cooling the exterior of the stainless-steel tubes with ice, an ultrasonic processor, the Sonifier 250 (made by Branson Corp.), was used to conduct a two-minute treatment at an output of 145 W. When 30 seconds had elapsed after the start of the ultrasonic treatment, an IC 3100 microsyringe pump (made by KD Scientific Corp.) was used to add 2 mL of 7.3 mM calcium chloride at a rate of 127 mL/hour. To the milk-white dispersion obtained was admixed 5 mL of 250 mPEU/mL PME solution and the mixture was reacted in a vibrator for four hours at a temperature of 40°C at pH 4. Subsequently, 1 mL of 7.3 mM calcium chloride was added to prepare a sample.
Measurement of the particle size distribution of the microcapsule dispersion with an LA920 laser light-scattering particle size distribution analyzer (made by Horiba Instruments) revealed a single peak, with the median outer diameter of the microcapsules being 1.8 µm.

### (Comparative Example 1)

### Preparation of a capsinoid emulsion with an ultrasonic device

Tween 20 (made by Sigma) and deionized water were mixed to prepare a 0.5 percent Tween 20 aqueous solution. Next, 27 mL of 0.5 percent Tween 20 solution and 3 mL of soybean oil (made by Ajinomoto Oil Mills) containing 2 percent capsinoids were added to a 50 mL stainless-steel tube. While cooling the exterior of the stainless-steel tube with ice, an ultrasonic processor, the Sonifier 250 (made by Branson Corp.), was used to conduct a two-minute treatment at an output of 145 W, yielding a milk-white dispersion.

Measurement of the particle size distribution of the emulsified dispersion with an LA920 laser light-scattering particle size distribution analyzer (made by Horiba Instruments) revealed a single peak, with the median diameter being 0.8 µm.

### (Embodiment 9)

### Evaluation of the heating stability of encapsulated capsinoids

The microcapsule dispersion obtained in Embodiment 8 and the emulsified dispersion obtained in Comparative Example 1 were each adjusted to pH 4 with concentrate hydrochloric acid and charged to glass containers (125 x 20 CV, made by Chromacol, Ltd.). The glass containers were placed in stainless-steel pressure-resistant reactors (TVS-N2-50, made by Taiatsu Techno Co., Ltd.), a pressure of 250 kPa was applied, and heating treatment was conducted for 30 minutes at 120°C to observe the change in capsinoid concentration. The results are given in Table 7. The microcapsule dispersion and emulsified dispersion were dissolved in ethyl acetate at a ratio of 1:1 and the capsinoid concentration was measured by high-performance liquid chromatography (column: J'sphere ODS-H80; mobile phase: 80 percent methanol, flow rate: 1 mL/min; temperature: 40°C; detector: fluorescence detector (Ex 280 nm, Em 320 nm)).

**[Table 7] Heating stability of microcapsules**

| Sample | Before heating | After heating: 120°C |
|---|---|---|
| Microcapsule dispersion | 100 | 82 |
| Emulsified dispersion | 100 | 59 |

In Table 7, the capsinoid concentration before heating is denoted as 100 percent and the capsinoid concentration after heating denotes the survival rate. From the results in Table 7, it will be understood that the microcapsule dispersion exhibited better heating stability of encapsulated capsinoids than the emulsified dispersion.

### (Embodiment 10)

### Evaluation of storage stability of encapsulated capsinoids

The microcapsule dispersion and emulsified dispersion of Embodiment 9 were pH-adjusted, heat treated, charged to sealed containers, and stored for 30 days at 5°C, 24°C, and 44°C to observe changes in capsinoid concentration. The results are given in Table 8.

**[Table 8] Storage stability of microcapsules**

| Sample | Before heating | After heating | Storage temperature | 10-day storage | 30-day storage |
|---|---|---|---|---|---|
| Microcapsule dispersion | 100 | 82 | 5 °C | 82 | 82 |
| | | | 24°C | 82 | 82 |
| | | | 44°C | 60 | 52 |
| Emulsified dispersion | 100 | 59 | 5°C | 59 | 59 |
| | | | 24°C | 40 | 40 |
| | | | 44°C | 18 | 13 |

### (Embodiment 11)

### Manufacturing of alginic acid capsules with an ultrasonic device

Alginic acid (medium viscosity A-2033, made by Sigma Corp.) and deionized water were mixed to prepare a 0.25 percent alginic acid aqueous solution. Next, 27 mL of the 0.25 percent alginic acid aqueous solution and 3 mL of soybean oil (made by Ajinomoto Oil Mills Co., Ltd.) were charged to a 50 mL stainless-steel tube. While cooling the exterior of the stainless-steel tube with ice, an ultrasonic processor, the Sonifier 250 (made by Branson Corp.), was used to conduct a two-minute treatment at an output of 148 W.
The above dispersions, in which the soybean oil had been fluorescent dyed with Nile Red and the alginic acid with Rhodamine, were observed with an LSMS10 confocal laser scanning microscope (made by Carl Zeiss Corp.) to confirm the formation of microcapsules encapsulating soybean oil. Measurement of the particle size distribution of the microcapsule dispersion with an LA920 laser light-scattering particle size distribution analyzer (made by Horiba Instruments) revealed a single peak, with the median outer diameter of the microcapsules being 1.5 µm. The results are shown in Fig. 2.

### (Embodiment 12)

### Evaluation of capsules employing alginic acid and a reinforcing agent

Alginic acid (medium viscosity A-2033, made by Sigma Corp.) was mixed with deionized water to prepare solutions of a variety of concentrations in the same manner as in Embodiment 1. These were then mixed with soybean oil and processed for 2 minutes with ultrasonic waves. When about 30 seconds had elapsed after the start of ultrasonic processing, reinforcing agents were added in a variety of concentrations to prepare microcapsules.

The stability (in the form of the particle size distribution) and particle size of the various samples prepared were evaluated. The following criteria were employed in the evaluation of the present invention.
Single peak, median diameter of 3 µm or less: ⊚
Single peak, median diameter of greater than 3 µm: ○
Capsules tended not to form: △
No capsule formation: x
The evaluation results are given in Table 10

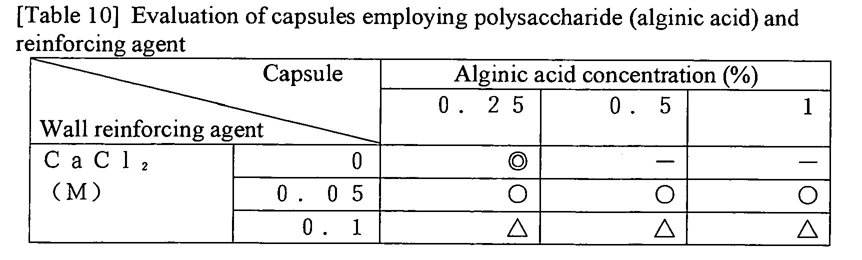

### (Embodiment 13)

### Preparation of capsinoid-containing microcapsules

Alginic acid (medium viscosity A-2033, made by Sigma Corp.) and deionized water were mixed to prepare a 1.0 percent alginic acid aqueous solution. A 27 mL quantity of this 1.0 percent alginic acid aqueous solution and 3 mL of soybean oil (made by Ajinomoto Oil Mills) containing 2 percent capsinoids (with a purity based on capsinoids of 97.5 percent and a capsiate to dihydrocapsiate ratio of about 2:1) were charged to a 50 mL stainless steel tube. While cooling the exterior of the stainless-steel tubes with ice, an ultrasonic processor, the Sonifier 250 (made by Branson Corp.), was used to conduct a two-minute treatment at an output of 148 W. When 30 seconds had elapsed after the start of the ultrasonic treatment, an IC 3100 microsyringe pump (made by KD Scientific Corp.) was used to add 2 mL of 50 mM calcium chloride at a rate of 127 mL/hour, yielding a milk-white dispersion.

The above dispersion, in which the soybean oil had been fluorescent dyed with Nile Red and the alginic acid with Rhodamine, was observed with an LSM510 confocal laser scanning microscope (made by Carl Zeiss Corp.) to confirm the formation of microcapsules encapsulating soybean oil. Measurement of the particle size distribution of the microcapsule dispersion with an LA920 laser light-scattering particle size distribution analyzer (made by Horiba Instruments) revealed a single peak, with the median outer diameter of the microcapsules being 5.3 µm.

### (Embodiment 14)

### Evaluation of the storage stability of encapsulated capsinoids

The microcapsule dispersion obtained in Embodiment 1 was adjusted to pH 4 with concentrate hydrochloric acid, charged to sealed containers, and stored for 30 days at 5°C, 24°C, and 44°C to observe the change in capsinoid concentration. The results are given in Table 1 [sic, should be 11]. The microcapsule dispersions were dissolved in a 1:1 ratio in ethyl acetate and the capsinoid concentration was measured by high-performance liquid chromatography (column: J'sphere ODS-H80; mobile phase: 80 percent methanol, flow rate: 1 mL/min; temperature: 40°C; detector: fluorescence detector (Ex 280 nm, Em 320 nm)).
The results are given in Table 11.

**[Table 11] Storage stability in microcapsule**

| Sample | Storage temperature | Before heating | 20-day storage | 30-day storage |
|---|---|---|---|---|
| Microcapsule dispersion | 5°C | 100 | 99 | 72 |
| | 24°C | | 100 | 75 |
| | 44°C | | 93 | 59 |

In Table 11, the capsinoid concentration before heating is denoted as 100 percent and the capsinoid concentration after heating denotes the survival rate. From the results in Table 1 [sic, should be 11], it will be understood that the microcapsule dispersion exhibited better storage stability of encapsulated capsinoids at 5°C and 24°C.

### (Reference Example)

Referring to the method described in Japanese Patent Application Publication No. 2000-312598, capsinoids enzymatically synthesized from starting materials in the form of vanillyl alcohol and fatty acid methyl obtained by refluxing capsinoids in wet methanolic hydrochloric acid were employed in the present invention. Specifically, synthesis was conducted with a 1:5 mole ratio of fatty acid methyl to vanillyl alcohol using enzyme in the form of immobilized lipase (Novozyme 435, made by Novozyme) in a 45-hour reaction at 25°C. The yield was 71.7 percent, the purity was 97.5 percent based on capsinoids, and the ratio of capsiate to dihydrocapsiate was 2:1.

## Claims

1. A capsinoid-containing microcapsule with a median diameter of 0.01 to 1,000 µm, comprising 0.01 to 10 weight parts of a capsinoid-containing hydrophobic material per weight part of a hydrophilic aqueous solution containing 0.001 to 50 percent of a capsule wall material.

2. The microcapsule according to claim 1, wherein said capsule wall material is one or more members selected from the group consisting of polysaccharides, proteins, and polyamino acids.

3. The microcapsule according to claim 1, wherein said capsule wall material is one or more members selected from the group consisting of alginic acid, pectin, and BSA.

4. The microcapsule according to any of claims 1 to 3, containing a pectin polyvalent cationic gel in the wall thereof, encapsulating a capsinoid-containing hydrophobic material, and having a median diameter of 0.01 to 100 µm, obtained by preparing an O/W emulsion with an aqueous phase containing 0.01 to 10 weight percent pectin and an oil phase and then admixing polyvalent cations.

5. The microcapsule according to claim 4, wherein said polyvalent cations are calcium ions.

6. The microcapsule according to claims 4 or 5, further **characterized in that** no emulsifier is employed when preparing the O/W emulsion.

7. The microcapsule according to any of claims 4 to 6, further **characterized in that** the pectin comprises an amide group.

8. The microcapsule according to any of claims 4 to 7, further **characterized in that** the hydrophobic material containing the encapsulated capsinoid is a W/O emulsion.

9. The microcapsule according to any of claims 1 to 8, further **characterized in that** pectin methyl esterase is added after preparing said O/W emulsion and admixing polyvalent cations.

10. The microcapsule according to claim 9, further **characterized in that** polyvalent cations are added after adding said pectin methyl esterase.

11. The microcapsule according to any of claims 1 to 10, further **characterized in that** said hydrophobic material containing the capsinoids is one or more selected from the group consisting of capsiates, dihydrocapsiates, or nordihydrocapsiates.

12. A method for manufacturing the microcapsule according to any of claims 1 to 3 with a median diameter of 0.01 to 1,000 µm, comprising ultrasonically treating for from 0.1 second to 60 minutes a mixed solution of 0.01 to 10 weight parts of capsinoid-containing hydrophobic material per weight part of hydrophilic solution containing 0.001 to 50 percent of capsule film material.

13. The method according to claim 12, wherein said capsule film material is alginic acid and calcium chloride is employed as a reinforcing agent.

14. The method according to claim 12, further **characterized in that** the capsule film material does not contain a reinforcing agent.

15. The method according to claim 12, further **characterized in that** the capsule film material does not contain an emulsifier.
